(19) Europäisches Patentamt
European Patent Office
Office européen des brevets

(11) **EP 4 159 855 A1**

(12) **EUROPEAN PATENT APPLICATION**
published in accordance with Art. 153(4) EPC

(43) Date of publication:
**05.04.2023 Bulletin 2023/14**

(21) Application number: **21813970.7**

(22) Date of filing: **19.05.2021**

(51) International Patent Classification (IPC):
**C12N 15/10** (2006.01)

(52) Cooperative Patent Classification (CPC):
**C12N 15/10**

(86) International application number:
**PCT/JP2021/019014**

(87) International publication number:
**WO 2021/241363 (02.12.2021 Gazette 2021/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.05.2020 JP 2020090405**

(71) Applicants:
• **Sekisui Medical Co., Ltd.**
**Tokyo 103-0027 (JP)**
• **Sekisui Chemical Co., Ltd.**
**Osaka-shi, Osaka 530-8565 (JP)**

(72) Inventors:
• **YAMAGUCHI, Sou**
**Tokyo 103-0027 (JP)**
• **UCHIDA, Katsura**
**Tokyo 103-0027 (JP)**
• **KOUNO, Takamasa**
**Mishima-gun, Osaka 618-0021 (JP)**
• **TAKAMATSU, Tatsunori**
**Tokyo 103-0027 (JP)**
• **KINOSHITA, Takuya**
**Mishima-gun, Osaka 618-0021 (JP)**

(74) Representative: **Ter Meer Steinmeister & Partner
Patentanwälte mbB
Nymphenburger Straße 4
80335 München (DE)**

(54) **METHOD FOR PURIFYING NUCLEIC ACID**

(57)    Provided is a method for purifying a nucleic acid capable of easily purifying a nucleic acid and efficiently recovering the nucleic acid. The method for purifying a nucleic acid including a step of bringing a sample containing a nucleic acid into contact with an extraction solution containing a metal cation to prepare an extraction solution containing the nucleic acid, a step of bringing the extraction solution containing the nucleic acid into contact with an anionic adsorbent 4 to cause the nucleic acid to adsorb to the anionic adsorbent 4, a step of bringing a wash solution having a pH of 5.0 or less into contact with the anionic adsorbent 4 to wash the anionic adsorbent 4 to which the nucleic acid is adsorbed, and a step of bringing a recovery solution having a pH of 6.0 or more into contact with the anionic adsorbent 4 to isolate the nucleic acid from the anionic adsorbent 4.

[FIG. 2.]

**Description**

**TECHNICAL FIELD**

The present invention relates to a method for purifying a nucleic acid.

**BACKGROUND ART**

Conventionally, as a method for purifying a nucleic acid such as RNA or DNA contained in a sample such as a virus, a bacterium, a fungus, or a cell, a method is known in which a precipitation reagent such as alcohol is added to an extraction liquid containing a nucleic acid derived from the sample to precipitate the nucleic acid, the precipitated nucleic acid is caused to adsorb to a solid phase carrier, impurities such as a protein derived from the sample attached to the solid phase carrier are washed together with the nucleic acid, and then the nucleic acid is recovered using an eluate. In such a method, an alcohol such as ethanol is usually used not only for the precipitation reagent but also for the extraction reagent and the wash solution.

For example, Patent Document 1 below discloses a method for extracting a nucleic acid by bringing a cell raw material into contact with an extraction solution to dissolve a cell material. In Patent Document 1, an alcohol such as ethanol or butanol is used for the extraction solution.

Patent Document 2 below discloses a method including bringing a solid phase to which a nucleic acid is bound into contact with a wash solution containing at least one component among components constituting a reaction liquid applicable to an enzyme reaction using the nucleic acid to wash the solid phase. In Examples of

Patent Document 2, an alcohol is used as a second wash solution.

**Related Art Document**

**Patent Documents**

Patent Document 1: JP 2018-134093 A
Patent Document 2: JP 4340298 B

**SUMMARY OF THE INVENTION**

**PROBLEMS TO BE SOLVED BY THE INVENTION**

However, when the extraction solution contains an alcohol as in Patent Document 1, the nucleic acid cannot be sufficiently extracted in some cased because the alcohol coexists when the nucleic acid is extracted from the sample. Thus, there is a problem that a step of mixing an alcohol for precipitating the nucleic acid after extracting the nucleic acid from the sample is required, and the process becomes complicated.

When the wash solution contains an alcohol as in Patent Document 2, it is necessary to pay attention to volatilization and ignition of the alcohol when the wash solution is stored, which causes a problem of poor handling characteristics. In addition, in Patent Document 2, a washing step is provided three times so as not to bring the alcohol into a subsequent step, which complicates the process.

When no alcohol is used, the recovery rate of the nucleic acid decreases, and the nucleic acid is not efficiently recovered in some cases. In addition, a substance used for nucleic acid purification other than alcohols may inhibit a reaction in testing or analysis in a subsequent step.

An object of the present invention is to provide a method for purifying a nucleic acid capable of easily purifying a nucleic acid and efficiently recovering the nucleic acid.

**MEANS FOR SOLVING THE PROBLEMS**

A method for purifying a nucleic acid includes a step of bringing a sample containing a nucleic acid into contact with an extraction solution containing a metal cation to prepare an extraction solution containing the nucleic acid, a step of bringing the extraction solution containing the nucleic acid into contact with an anionic adsorbent to cause the nucleic acid to adsorb to the anionic adsorbent, a step of bringing a wash solution having a pH of 5.0 or less into contact with the anionic adsorbent to wash the anionic adsorbent to which the nucleic acid is adsorbed, and a step of bringing a recovery solution having a pH of 6.0 or more into contact with the anionic adsorbent to isolate the nucleic acid from the anionic adsorbent.

In a specific aspect of the method for purifying a nucleic acid according to the present invention, the recovery solution is a solution that does not inhibit a nucleic acid amplification reaction. The recovery solution is preferably a buffer solution. The concentration of the buffer solution is more preferably 5 mmol/L or more and 100 mmol/L or less. The recovery solution may be a solution containing a nucleic acid amplification reagent.

In another specific aspect of the method for purifying a nucleic acid according to the present invention, the wash solution having a pH of 5.0 or less is at least one selected from the group consisting of water, a hydrochloric acid-potassium chloride buffer solution, a glycine-hydrochloric acid buffer solution, a citric acid-sodium citrate buffer solution, and a citric acid-phosphate buffer solution.

In still another specific aspect of the method for purifying a nucleic acid according to the present invention, the concentration of the metal cation in the extraction solution is 0.5 mol/L or more.

In still another specific aspect of the method for purifying a nucleic acid according to the present invention, the metal cation is an alkali metal ion, an alkaline earth metal ion, a transition metal ion, or a group 12 metal ion.

In still another specific aspect of the method for purifying a nucleic acid according to the present invention, the metal cation has a valence of 2 or more.

## EFFECT OF THE INVENTION

The present invention can provide a method for purifying a nucleic acid capable of easily purifying a nucleic acid and efficiently recovering the nucleic acid.

## BRIEF DESCRIPTION OF DRAWINGS

[Fig.1] Fig. 1 is a schematic view for explaining a state in which a nucleic acid is adsorbed to an anionic adsorbent in a method for purifying a nucleic acid according to an embodiment of the present invention.

[Fig.2] Fig. 2 is a schematic plan view illustrating a chip used in the method for purifying a nucleic acid according to an embodiment of the present invention.

[Fig.3] Fig. 3 is a schematic sectional view of a part taken along the line A-A in Fig. 2.

[Fig.4] Fig. 4 is a graph showing the relationship between the pH of a wash solution and the proportion of RNA in the wash solution and a recovery solution.

[Fig.5] Fig. 5 is a graph showing the relationship between the pH of a wash solution and the recovery rate of RNA.

[Fig.6] Fig. 6 is a graph showing the relationship between the pH of a wash solution and the recovery rate of RNA when nasal mucus is added.

[Fig.7] Fig. 7 is a diagram showing the relationship between the number of PCR cycles and the fluorescence intensity when a potassium chloride-hydrochloric acid buffer solution having a pH of 2 is used as a wash solution in Example 3.

[Fig.8] Fig. 8 is a diagram showing the relationship between the number of PCR cycles and the fluorescence intensity when water having a pH of 2 is used as a wash solution in Example 6.

[Fig.9] Fig. 9 is a graph showing the relationship between the type of metal cation and the recovery rate of RNA.

## MODES FOR CARRYING OUT THE INVENTION

Hereinafter, the present invention will be clarified by describing specific embodiments of the present invention with reference to the drawings.

In a method for purifying a nucleic acid according to the present invention, first, a sample containing a nucleic acid is brought into contact with an extraction solution containing a metal cation to prepare an extraction solution containing the nucleic acid (extraction step). Next, the extraction solution containing the nucleic acid is brought into contact with an anionic adsorbent to cause the nucleic acid to adsorb to the anionic adsorbent (nucleic acid adsorption step). Next, a wash solution having a pH of 5.0 or less is brought into contact with the anionic adsorbent to wash the anionic adsorbent to which the nucleic acid is adsorbed (washing step). Next, a recovery solution having a pH of 6.0 or more is brought into contact with the anionic adsorbent to isolate the nucleic acid from the anionic adsorbent (nucleic acid recovery step).

The method for purifying a nucleic acid of the present invention enables a nucleic acid to be easily purified and efficiently recovered. The reason for this can be described as follows.

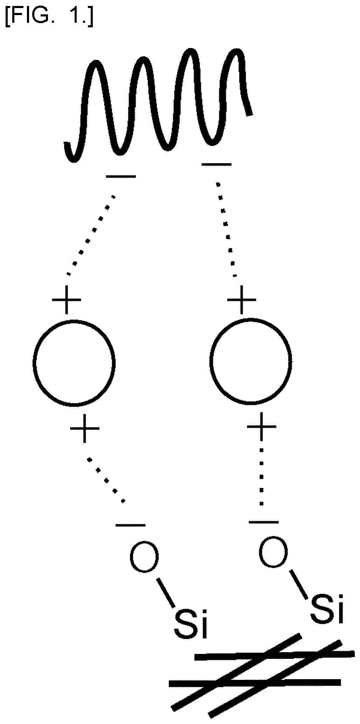

In the present invention, the extraction solution used in the extraction step contains a metal cation. Thus, when the sample containing a nucleic acid contacts the extraction solution, the metal cation binds ionically to the nucleic acid that is negatively charged. When such an extraction solution after the nucleic acid is extracted is brought into contact with the anionic adsorbent, the metal cation binds ionically to the anionic adsorbent this time as illustrated in Fig. 1. The nucleic acid can thus adsorb to the anionic adsorbent via the metal cation.

In this manner, in the present invention, it is possible to easily cause the nucleic acid to adsorb to the anionic adsorbent via the metal cation. Since the metal cation does not inhibit the extraction of the nucleic acid even when the metal cation is added to the nucleic acid extraction solution, a step of further adding a solution does not have to be provided after the nucleic acid extraction unlike in the conventional method using an alcohol. Thus, a complicated process is not required.

In addition, the present invention can cause a nucleic acid to adsorb to an anionic adsorbent without an alcohol, which improves handleability. Further, when a large amount of alcohol is added to precipitate a nucleic acid, the volume of the extraction solution after the nucleic acid extraction increases and the concentration of the nucleic acid decreases, whereas in the nucleic acid purification using a metal cation in the present invention, the volume of the extraction solution does not increase (the nucleic acid concentration does not decrease), which can increase the recovery rate of the nucleic acid. As a result, enough nucleic acid can be obtained in one purification treatment, which can improve the sensitivity of analysis and testing in the subsequent steps.

When a solution containing a large amount of metal cations is used in a nucleic acid amplification step in the subsequent step, nucleic acid amplification may be inhibited. Thus, in the washing step, it is necessary to wash away the excess metal cations bound to the anionic adsorbent with the nucleic acid. However, when a solution containing no alcohol is used as the wash solution, the nucleic acid may desorb from the anionic adsorbent together with the metal cations.

In the present invention, by using a wash solution having a pH of 5.0 or less, it is possible to wash away excess metal cations without desorbing the nucleic acid bound to the anionic adsorbent. Thus, the nucleic acid can be efficiently recovered, and moreover, nucleic acid amplification is hardly inhibited by the metal cation in the subsequent step, which can improve the accuracy of analysis and testing. In addition, since the nucleic acid can be reliably washed at one washing without using an alcohol, the nucleic acid can be easily purified with excellent handleability. In the present invention, a recovery solution having a pH of 6.0 or more is used, and because of this too, the nucleic acid can be efficiently recovered. Moreover, the nucleic acid amplification is hardly inhibited by the metal cation in the subsequent step, which can improve the accuracy of analysis and testing.

Hereinafter, each step will be described in more detail.

(Extraction step)

In the extraction step, a sample containing a nucleic acid is brought into contact with an extraction solution. This causes the nucleic acid in the sample to be extracted, whereby an extraction solution containing the nucleic acid is obtained.

Examples of the sample containing a nucleic acid include a biological sample containing a nucleic acid such as DNA or RNA. Examples of such a biological sample include cells, blood, tissue fluid, urine, and feces. Examples of the sample containing a nucleic acid include a sample containing a nucleic acid in the environment such as soil, sea water, or river water, and is not particularly limited.

As the extraction solution, for example, a solution containing a protein denaturant, a metal cation, and a polar solvent may be used. The polar solvent is preferably contained in an amount of 50% or more in the extraction solution.

The protein denaturant has a role of breaking down a higher order structure of a protein by interacting with the protein. Examples of the protein denaturant that may be used include a surfactant, a reducing agent, a guanidine derivative, thiourea, urea, and a salt thereof. Examples of the surfactant that may be used include sodium dodecyl sulfate (SDS) and polyoxyethylene sorbitan monolaurate (Tween 20). Examples of the reducing agent that may be used include 2-mercaptoethanol and dithiothreitol (DTT). The examples of the salt that may be used include guanidine hydrochloride. The protein denaturants described above may be used singly or in combination of two or more thereof.

The concentration of the protein denaturant in the extraction solution is not particularly limited, but is preferably 2 mol/L or more, more preferably 4 mol/L or more, still more preferably 8 mol/L or more, and preferably 10 mol/L or less. Setting the concentration of the protein denaturant within the above range enables the nucleic acid to be extracted with increased reliability. When two or more protein denaturants are contained, it is preferable that the total concentration be within the above range.

The metal cation may be used without particular limitation, and example thereof include an alkali metal ion, an alkaline earth metal ion, a transition metal ion, and a group 12 metal ion.

Examples of the alkali metal ion include the potassium ion, the sodium ion, the lithium ion, the rubidium ion, the cesium ion, and the francium ion.

Examples of the alkaline earth metal ion include the calcium ion, the magnesium ion, the beryllium ion, the strontium ion, the barium ion, and the radium ion.

Examples of the transition metal ion include the manganese ion, the iron ion, the cobalt ion, the nickel ion, and the copper ion.

Examples of the group 12 metal ion include the zinc ion, the cadmium ion, and the mercury ion.

These metal cations may be used singly or in combination of two or more thereof.

The metal cation preferably has a valence of 2 or more. Examples of the metal cation having a valence of 2 or more include the calcium ion and the magnesium ion. Using a metal cation having a valence of 2 or more enables the nucleic acid to be recovered with increased efficiency.

The concentration of the metal cation in the extraction solution is preferably 0.5 mol/L or more, more preferably 1 mol/L or more, and still more preferably 2 mol/L or more. When the concentration of the metal cation is the above-described lower limit value or more, it is possible to recover the nucleic acid with increased efficiency. The upper limit value of the concentration of the metal cation is not particularly limited, and may be, for example, 6 mol/L.

The polar solvent may be used without particular limitation, and examples thereof include water, dimethyl sulfoxide (DMSO), N,N-dimethylformamide (DMF), dimethylacetamide (DMA), methoxypropanol, polyethylene glycol, pentanediol, propanediol, aminoethanol, and diethanolamine. These polar solvents may be used singly or in combination of two or more thereof. The extraction solution does not have to contain a polar solvent. The extraction solution may contain a solvent other than a polar solvent. From the viewpoint of extracting a nucleic acid with increased reliability, it is preferable that the extraction solution contain a polar solvent such as water.

The extraction solution may further contain a coprecipitation agent. Examples of the coprecipitation agent include tRNA, polyadenine, an acrylamide polymer, and glycogen. The extraction solution further containing a coprecipitation agent enables the nucleic acid to adsorb to the anionic adsorbent with increased efficiency.

(Nucleic acid adsorption step)

In the nucleic acid adsorption step, the extraction solution containing the nucleic acid is brought into contact with an anionic adsorbent to cause the nucleic acid to adsorb to the anionic adsorbent.

The anionic adsorbent is a carrier for carrying the nucleic acid. The anionic adsorbent that may be used is not limited to particular forms, and examples of the form include a film, a filter, a plate, a tube, and a fiber form. The shape of the anionic adsorbent is preferably a fiber shape, a particle shape, or a porous shape.

The anionic adsorbent is not particularly limited, and may be composed of, for example, a silicon compound, a phosphate mineral, a silicate mineral, or an aluminosilicate mineral. Examples of the silicon compound include silica and glass. Examples of the phosphate mineral include hydroxyapatite. Examples of the silicate mineral include talc and montmorillonite. Examples of the aluminosilicate mineral include zeolite. These anionic adsorbents may be used singly or in combination of two or more thereof.

The anionic adsorbent is preferably silica fibers or glass fibers, and more preferably silica fibers. In the present embodiment, silica fibers are used as the anionic adsorbent. However, the anionic adsorbent may be silica particles or a porous body of silica.

(Washing step)

In the washing step, a wash solution having a pH of 5.0 or less is brought into contact with the anionic adsorbent to which the nucleic acid has adsorbed in the nucleic acid adsorption step to wash the anionic adsorbent to which the nucleic acid is adsorbed.

Examples of the wash solution having a pH of 5.0 or less include water, a hydrochloric acid-potassium chloride buffer solution, a glycine-hydrochloric acid buffer solution, a citric acid-sodium citrate buffer solution, and a citric acid-phosphate buffer solution. These wash solutions may be used singly or in combination of two or more thereof.

The pH of the wash solution is 5.0 or less, preferably 4.0 or less. When the pH of the wash solution is the above-described upper limit value or less, it is possible to remove unnecessary metal ions with increased reliability without desorbing the nucleic acid bound to the anionic adsorbent and to make the amplification of the nucleic acid in the subsequent step less likely to be inhibited. In addition, since an alcohol which inhibits the nucleic acid amplification reaction is not used in the wash solution, there is no need of an operation to remove an alcohol before the nucleic acid amplification reaction, and the nucleic acid can be purified more easily.

The lower limit value of the pH of the wash solution may be, for example, 2.0.

(Nucleic acid recovery step)

Next, the anionic adsorbent washed in the washing step is brought into contact with a recovery solution to isolate the nucleic acid from the anionic adsorbent and recover the nucleic acid.

The recovery solution is preferably a solution that does not inhibit the nucleic acid amplification reaction. In this case, it is possible to further increase the accuracy of analysis and testing in the subsequent step. The pH of the recovery solution is 6.0 or more, preferably 7.0 or more, and more preferably 8.0 or more. In this case, the nucleic acid can be recovered with increased efficiency. The upper limit value of the pH of the recovery solution is not particularly limited, and may be, for example, 9.0.

As the recovery solution, a buffer solution, a nucleic acid amplification reagent, or the like may be used.

Examples of the buffer solution that may be used include trishydroxymethylaminomethane (Tris), N-tris(hydroxymethyl)methyl-2-aminoethanesulfonic acid (TES), tricine, PIPES, ACES, MOPSO, BES, MOPS, HEPES, TAPSO, POPSO, HEPSO, EPPS, Bicine, TAPS, and a phosphate buffer solution.

Examples of the reagent for nucleic acid amplification that may be used include TaqPath 1-Step Multiplex MasterMix, TaqMan Gene Expression Master Mix, TaqMan Fast Advanced MasterMix Mix, TaqPath qPCR Master Mix, CG, and TaqMan Fast Virus 1-Step Master Mix (manufactured by Thermo Fisher Scientific).

These may be used singly or in combination of two or more thereof.

The pH of the buffer solution is 6.0 or more, preferably 7.0 or more, and more preferably 8.0 or more. In this case, the nucleic acid can be recovered with increased efficiency. The upper limit value of the pH of the buffer solution is not particularly limited, and may be, for example, 9.0.

The concentration of the buffer solution is preferably 5 mmol/L or more, more preferably 10 mmol/L or more, and preferably 100 mmol/L or less, more preferably 50 mmol/L or less, further preferably 30 mmol/L or less. When the concentration of the buffer solution is within the above-described range, the nucleic acid can be recovered with increased efficiency.

Hereinafter, more specific embodiments will be exemplified.

(First embodiment)

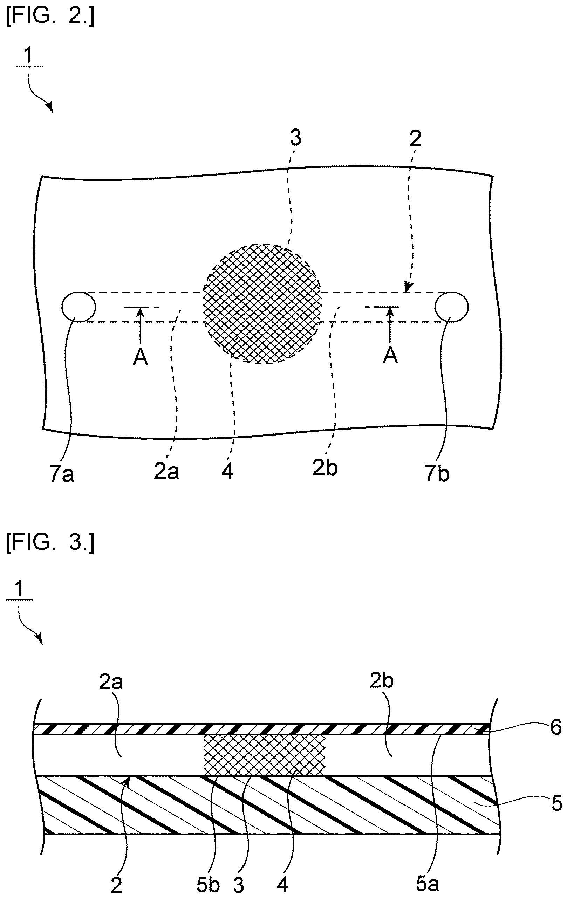

Fig. 2 is a schematic plan view illustrating a chip used in a method for purifying a nucleic acid according to a first embodiment of the present invention. Fig. 3 is a schematic sectional view of a part taken along the line A-A in Fig. 2.

As illustrated in Figs. 2 and 3, a chip 1 has a flow path 2 through which a fluid is sent. A recovery part 3 is provided in the middle of the flow path 2. The flow path 2 therefore includes an upstream flow path 2a provided upstream of the recovery part 3 and a downstream flow path 2b provided downstream of the recovery part 3. An anionic adsorbent 4 for adsorbing and purifying a nucleic acid is disposed in the recovery part 3.

The chip 1 is a chip used for testing and analysis. The chip 1 is not particularly limited, but in the present embodiment, it includes a plate-like substrate 5 and a cover member 6 as illustrated in Fig. 3. The substrate 5 has a main face 5a. The substrate 5 is provided with a recess 5b on the main face 5a side. The recess 5b is provided to be open to the main face 5a side of the substrate 5.

The material constituting the substrate 5 is not particularly limited, and for example, synthetic resin, rubber, metal, or the like may be used. The synthetic resin is not particularly limited and is preferably a thermoplastic resin. In particular, as the thermoplastic resin, for example, a cycloolefin polymer, a cycloolefin copolymer, polycarbonate, polymethyl methacrylate, polypropylene, or the like may be used. These resins may be used singly or in combination of two or more thereof.

The substrate 5 is preferably formed of a molded body of the thermoplastic resin. The molding method is not particularly limited, and a known molding method may be used. Examples of the molding method include injection molding, injection compression molding, gas assist method injection molding, extrusion molding, multilayer extrusion molding, rotation molding, hot press molding, blow molding, and foam molding. Among them, injection molding is preferable.

The substrate 5 may be formed by stacking a plurality of synthetic resin sheets. The substrate 5 may include a base sheet and a substrate body having a through hole provided on the base sheet.

The substrate 5 is provided with a cover member 6 on the main face 5a. The cover member 6 is provided to close the recess 5b of the substrate 5. The cover member 6 closes the recess 5b of the substrate 5 to form the recovery part 3. In the present embodiment, the upstream flow path 2a and the downstream flow path 2b are similarly configured by the cover member 6 closing the recess 5b of the substrate 5.

The cover member 6 may be composed of, for example, a flexible material such as a resin film. As the resin film, for example, a thermoplastic resin such as a cycloolefin polymer, a cycloolefin copolymer, polycarbonate, polymethyl methacrylate, or polypropylene may be used.

The cover member 6 may be composed of an elastic member. The elastic member is not particularly limited and is preferably an elastomer. In the present invention, the substrate 5 and the cover member 6 may be integrally formed.

In the substrate 5, the flow path 2 described above through which a fluid is sent is provided. Here, the flow path 2 is a micro flow path. The flow path 2 may be a flow path having a sectional area larger than that of a micro flow path, instead of a micro flow path. However, the flow path 2 is preferably a micro flow path. This enables various analyses to be performed with a trace amount of sample.

The micro flow path refers to a fine flow path in which a micro effect is generated when a fluid is transferred. In such a micro flow path, a liquid is strongly affected by surface tension, and exhibits behavior different from that of the liquid flowing through a flow path having a normal large size.

The transverse sectional shape and size of the micro flow path are not particularly limited as long as the micro flow path is a flow path in which the above-described micro effect occurs. For example, when a pump or gravity is used for causing a fluid to flow in the micro flow path and the micro flow path has a substantially rectangular (including square) transverse sectional shape, the length of a smaller side of the transverse sectional shape is preferably 20 um or more, more preferably 50 um or more, still more preferably 100 $\mu$m or more from the viewpoint of reducing the channel resistance. From the viewpoint of further downsizing of a microfluidic device using the chip 1, the length of the smaller side is preferably 5 mm or less, more preferably 1 mm or less, still more preferably 500 um or less.

When the micro flow path has a substantially circular transverse sectional shape, the diameter (minor axis in the case of ellipse) of the circle is preferably 20 um or more, more preferably 50 um or more, still more preferably 100 um or more. From the viewpoint of further downsizing the microfluidic device, the diameter (minor axis in the case of ellipse) is preferably 5 mm or less, more preferably 1 mm or less, still more preferably 500 um or less.

In the present embodiment, the above-described method for purifying a nucleic acid according to the present invention is performed using such a chip 1.

Specifically, the extraction solution after the nucleic acid extraction in the extraction step for the nucleic acid is injected into the upstream flow path 2a from an injection port 7a in the chip 1 and sent to the recovery part 3. Whereby, the nucleic acid is caused to adsorb to the anionic adsorbent 4 in the recovery part 3.

Next, the wash solution having a pH of 5.0 or less is sent to the recovery part 3. Whereby, the anionic adsorbent 4 to which the nucleic acid is adsorbed is washed. Next, the recovery solution is sent to the recovery part 3 to cause the nucleic acid to be sent to the downstream flow path 2b side while being isolated from the anionic adsorbent 4, then the nucleic acid is recovered from the recovery port 7b.

In the method for purifying a nucleic acid of the present embodiment, it is possible to easily cause the nucleic acid to adsorb to the anionic adsorbent 4 via the metal cation. Since the metal cation does not inhibit the extraction of the nucleic acid even when the metal cation is added to the extraction solution before the extraction of the nucleic acid, a step of adding an alcohol does not have to be provided after the extraction unlike in the conventional method using an alcohol. Thus, a complicated process is not required.

In addition, the present invention enables the nucleic acid to adsorb to the anionic adsorbent 4 without using an alcohol, which improves handleability. Further, when a large amount of alcohol is added to precipitate a nucleic acid, the volume of the extraction solution after the nucleic acid extraction increases and the concentration of the nucleic acid decreases, whereas in the nucleic acid purification using a metal cation in the present invention, the volume of the extraction solution does not increase (the nucleic acid concentration does not decrease), which can increase the recovery rate of the nucleic acid. As a result, enough nucleic acid can be obtained in one purification treatment, which can improve the sensitivity of analysis and testing in the subsequent steps.

When a solution containing a large amount of metal cations is used in a nucleic acid amplification step in the subsequent step, nucleic acid amplification may be inhibited. Thus, in the washing step, it is necessary to wash away excess metal cations bound to the anionic adsorbent with the nucleic acid. However, when a solution containing no alcohol is used as the wash solution, the nucleic acid may desorb from the anionic adsorbent together with the metal cations.

In the present invention, by using a wash solution having a pH of 5.0 or less, it is possible to wash away excess metal cations without desorbing the nucleic acid bound to the anionic adsorbent. Thus, in the subsequent step, nucleic acid amplification is hardly inhibited by the metal cation, which can increase the accuracy of analysis and testing. In addition, since the nucleic acid can be reliably washed at one washing without using an alcohol, the nucleic acid can be easily purified with excellent handleability.

(Second embodiment)

In a second embodiment, the above-described method for purifying a nucleic acid according to the present invention is performed using a container.

Specifically, first, an anionic adsorbent for adsorbing and recovering a nucleic acid is disposed in a container made of plastic such as polypropylene. The extraction solution after the nucleic acid extraction in the extraction step for the nucleic acid is injected into the container and stirred with the anionic adsorbent to cause the nucleic acid to adsorb to the anionic adsorbent. The stirring method is not particularly limited, and for example, a vortex mixer, an ultrasonic wave, or the like may be used.

Next, after the solution in the container is taken out, a wash solution is injected and stirred. Whereby, the anionic adsorbent to which the nucleic acid has adsorbed is washed. Next, after the wash solution is taken out, a recovery solution is injected and stirred. Whereby, it is possible to isolate the nucleic acid from the anionic adsorbent. Finally, the recovery solution is taken out, and the isolated nucleic acid is recovered.

The extraction solution contains a metal cation also in the second embodiment. Thus, when the sample containing a nucleic acid is added to the extraction solution, the metal cation binds ionically to the nucleic acid that is negatively charged. When the mixed solution after extraction of the nucleic acid to which the metal cation is ionically bound is brought into contact with the anionic adsorbent, the metal cation binds ionically to the anionic adsorbent. The nucleic acid can thus adsorb to the anionic adsorbent via the metal cation.

In this manner, in the purification method of the present embodiment, it is possible to easily cause the nucleic acid to adsorb to the anionic adsorbent via the metal cation. Since the metal cation does not inhibit the extraction of the nucleic acid even when the metal cation is added to the extraction solution before the extraction of the nucleic acid, a step of adding an alcohol does not have to be provided after the extraction unlike in the conventional method using an alcohol. Thus, a complicated process is not required.

In addition, the present invention can cause a nucleic acid to adsorb to an anionic adsorbent without using an alcohol, which improves handleability. Further, when a large amount of alcohol is added to precipitate a nucleic acid, the volume of the extraction solution after the nucleic acid extraction increases and the concentration of the nucleic acid decreases, whereas in the nucleic acid purification using a metal cation in the present invention, the volume of the extraction solution does not increase (the nucleic acid concentration does not decrease), which can increase the recovery rate of the nucleic acid. As a result, enough nucleic acid can be obtained in one purification treatment, which can improve the sensitivity of analysis and testing in the subsequent steps.

When a solution containing a large amount of metal cations is used in a nucleic acid amplification step in the subsequent step, nucleic acid amplification may be inhibited. Thus, in the washing step, it is necessary to wash away excess metal cations bound to the anionic adsorbent with the nucleic acid. However, when a solution containing no alcohol is used as the wash solution, the nucleic acid may desorb from the anionic adsorbent together with the metal cations.

In the present invention, by using a wash solution having a pH of 5.0 or less, it is possible to wash away excess metal cations without desorbing the nucleic acid bound to the anionic adsorbent. Thus, in the subsequent step, nucleic acid amplification is hardly inhibited by the metal cation, which can increase the accuracy of analysis and testing. In addition, since the nucleic acid can be reliably washed at one washing without using an alcohol, the nucleic acid can be easily purified with excellent handleability.

Hereinafter, the present invention will be clarified with reference to specific Examples and Comparative Examples of the present invention. The present invention is not limited to the following Examples.

(Examples 1 to 2 and Comparative Examples 1 to 4)

In Examples 1 to 2 and Comparative Examples 1 to 4, the chip 1 illustrated in Figs. 2 and 3 was produced as follows.

A cycloolefin polymer was used as the material constituting the substrate 5. The cycloolefin polymer was subjected to injection molding, whereby the substrate 5 having the recess 5b was produced. A sealing tape was used for the cover member 6. The recess 5b of the substrate 5 was closed with the sealing tape, whereby the chip 1 was produced. In the recovery part 3, silica fibers (2 mmφ, thickness 0.8 mm) were disposed as the anionic adsorbent 4. The width of the flow path 2 was 0.8 mm, and the depth was 0.8 mm.

The recovery rate was measured as follows with such a chip 1.

First, 1 μL of a sample containing a nucleic acid (RNA) (virus; 30,000 copies) was added to 150 μL of an extraction solution (aqueous solution containing 4 mol/L of urea, 4 mol/L of guanidine hydrochloride, 2 mol/L of calcium chloride, and Tris-HCl Buffer (pH 7.0)) to extract the nucleic acid.

Next, the extraction solution after the nucleic acid extraction was sent from the upstream flow path 2a to the recovery part 3 to cause the nucleic acid to adsorb to the silica fibers of the recovery part 3.

Next, 400 μL of a wash solution was sent to the recovery part 3, the silica fibers carrying the nucleic acid were washed, and the wash solution after sent was recovered. A recovery solution (10 mmol/L, Tris-HCl Buffer (pH 8.0)) (21 μL) was sent to the recovery part 3, and the nucleic acid (RNA) supported by the recovery part 3 was isolated and recovered.

In Example 1, water having a pH of 4.0 was used as the wash solution. In Example 2, water having a pH of 5.0 was used. In Comparative Example 1, a buffer solution (Tris) having a concentration of 10 mmol/L and a pH of 6.0 was used. In Comparative Example 2, a buffer solution (Tris) having a concentration of 10 mmol/L and a pH of 7.0 was used. In Comparative Example 3, a buffer solution (Tris) having a concentration of 10 mmol/L and a pH of 8.0 was used. In Comparative Example 4, a buffer solution (Tris) having a concentration of 10 mmol/L and a pH of 9.0 was used.

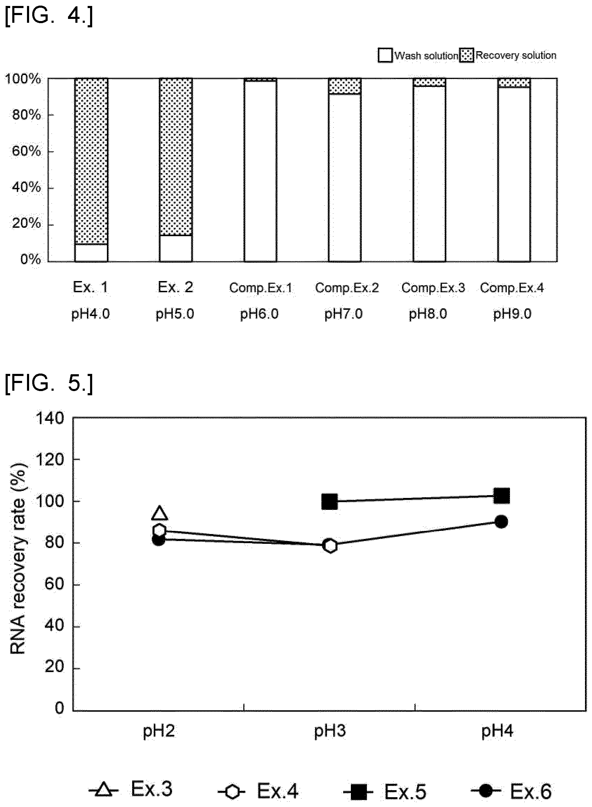

Next, the proportion of RNA in the recovered wash solution and the recovery solution was evaluated. First, RNA in the wash solution after sent was purified with QIAamp 96 Virus QIAcube HT Kit (manufactured by QIAGEN) and QIAcube HT System (manufactured by QIAGEN) (purified wash solution). Subsequently, a RT-PCR reaction solution was prepared using the RNA solution purified from the wash solution or 2 μL of the recovery solution, a primer, and TaqPath 1-Step Multiplex MasterMix (manufactured by Thermo Fisher Scientific). As a standard, an RT-PCR reaction solution (3 levels of nucleic acid concentration of 50,000 · 5,000 · 500 copies/μL) containing 2 μL of a solution obtained by extracting and purifying the same virus using QIAamp Viral RNA Mini Kit manufactured by QIAGEN was also prepared. Next, the RT-PCR reaction solution prepared from the recovery solution and the standard RT-PCR reaction solution were amplified using a thermal cycler "CFX 96" manufactured by Bio-Rad Laboratories, Inc. For amplification, a reverse transcription reaction was performed at 50°C for 30 seconds, then initial denaturation was performed at 95°C for 20 seconds, and a PCR cycle was performed 45 times at 95°C for 3 seconds and 60°C for 5 seconds. After the amplification, the RNA recovery rate (nucleic acid recovery rate) was calculated using the following calculation formula from the amount of nucleic acid obtained by automatic calculation with CFX 96 from the standard. The nucleic acid recovery rate is desirably 5% or more to ensure analysis accuracy by nucleic acid amplification in the subsequent step. The results are shown in Fig. 4.

```
Nucleic acid recovery rate (%) = {(amount of nucleic acid

calculated by CFX 96 × amount of recovery solution/2) × 100}/3,0000
```

As shown in Fig. 4, it is found that in Examples 1 and 2 in which the pH is 5.0 or less, RNA can be inhibited from flowing out to the wash solution, and the nucleic acid can be recovered at a good recovery rate, as compared with Comparative Examples 1 to 4 in which the pH is more than 5.0. In Comparative Examples 1 to 4, the pH was adjusted using a buffer solution instead of water without adding metal cations such as sodium ions in consideration of the influence of metal cations.

(Examples 3 to 6)

In Example 3, the nucleic acid was recovered in the same manner as in Example 1 except that a hydrochloric acid-potassium chloride buffer solution having a concentration of 10 mmol/L and a pH of 2.0 was used as the wash solution. In Example 4, the nucleic acid was recovered in the same manner as in Example 1 except that a glycine-hydrochloric acid buffer solution having a concentration of 10 mmol/L and a pH of 2.0 or 3.0 was used as the wash solution. In Example 5, the nucleic acid was recovered in the same manner as in Example 1 except that a citric acid-sodium citrate buffer solution having a concentration of 10 mmol/L and a pH of 3.0 or 4.0 was used as the wash solution. In Example 6, the nucleic acid was recovered in the same manner as in Example 1 except that water (water-HCl) having a pH of 2.0, 3.0, or 4.0 was used as the wash solution. Subsequently, the recovery rate of the RNA recovered in the recovery solution was calculated in the same manner as in Example 1. The results are shown in Fig. 5.

As is apparent from Fig. 5, in Examples 3 to 6, RNA can be recovered at a good recovery rate.

For each sample, the relationship between the number of PCR cycles and the fluorescence intensity was determined.

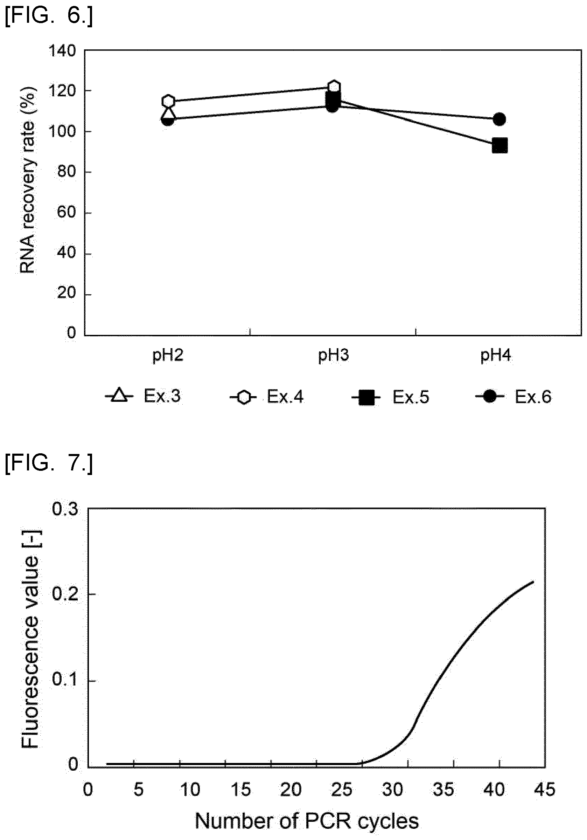

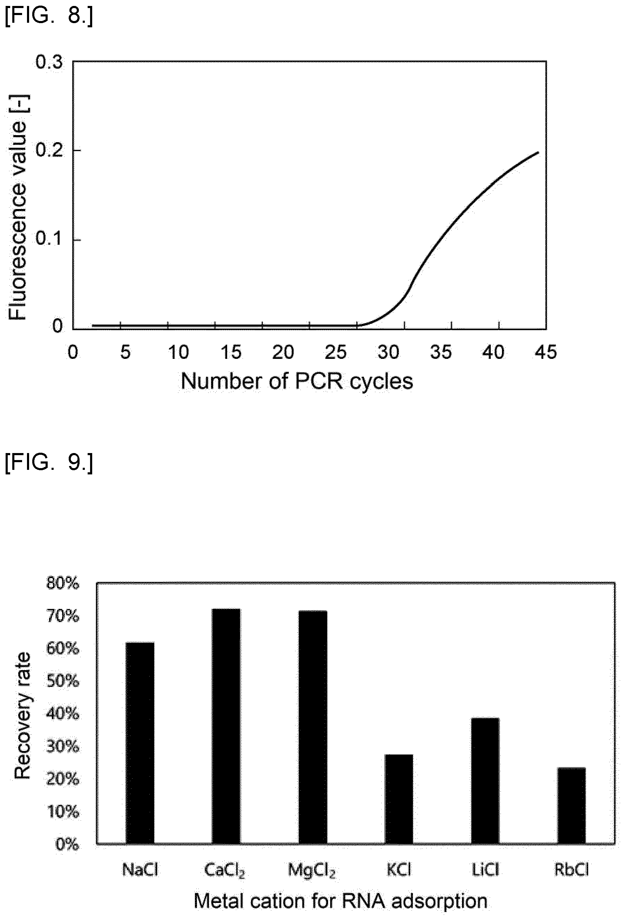

Fig. 7 is a diagram showing the relationship between the number of PCR cycles and the fluorescence intensity when a hydrochloric acid-potassium chloride buffer solution having a pH of 2.0 was used as the washing solution in Example 3. Fig. 8 is a diagram showing the relationship between the number of PCR cycles and the fluorescence intensity when water having a pH of 2.0 was used as the wash solution in Example 6.

As is apparent from the comparison between Fig. 7 and Fig. 8, even when a potassium chloride-hydrochloric acid buffer solution having a pH of 2.0 was used as the wash solution, the fluorescence intensity and the Ct value (the number of cycles at which the amplification curve started to rise) were almost the same as those when water having a pH of 2.0 was used as the wash solution. This shows that the nucleic acid amplification reaction is not inhibited even when a hydrochloric acid-potassium chloride buffer solution having a pH of 2.0 is used as the wash solution. In the same manner, the Ct value in each Example was determined. The results are shown in Table 1 below. The recovery rate and the Ct value in Table 1 below are average values of three times of measurement with N = 3. As for water, the result of using water having a pH of 5.0 as the wash solution is also shown. For comparison, the result of using a buffer solution (Tris-HCl) having a pH of 6.0 as the wash solution is also shown as Comparative Example 5.

[Table 1]

|  | Composition of wash solution | pH of wash solution | Recovery rate | Ct value |
|---|---|---|---|---|
| Ex. 3 | KCl - HCl | 2.0 | 93.4% | 32.01 |
| Ex. 4 | Glycine-HCl | 2.0 | 86.7% | 32.23 |
|  | Glycine-HCl | 3.0 | 78.9% | 32.34 |

(continued)

|  | Composition of wash solution | pH of wash solution | Recovery rate | Ct value |
|---|---|---|---|---|
| Ex. 5 | Citric acid-sodium citrate | 3.0 | 100.2% | 31.88 |
|  | Citric acid-sodium citrate | 4.0 | 103.4% | 31.83 |
| Ex. 6 | Water | 2.0 | 82.9% | 32.29 |
|  | Water | 3.0 | 78.9% | 32.32 |
|  | Water | 4.0 | 91.4% | 32.05 |
|  | Water | 5.0 | 24.2% | 33.54 |
| Comp.Ex.5 | Tris-HCl | 6.0 | 0.3% | 39.12 |

As shown in Table 1, in Examples 3 to 6, the nucleic acid recovery rate is 5% or more, which shows that the nucleic acid can be recovered well. In Examples 3 to 6, the Ct value is less than 38.0, and a remarkable increase in the Ct value is not observed, which shows that the nucleic acid amplification reaction is hardly inhibited. On the other hand, in Comparative Example 5, the nucleic acid recovery rate is less than 5%, which shows that the nucleic acid recovery rate is insufficient. In addition, in Comparative Example 5, the Ct value is 38.0 or more with a decreased recovery rate, which shows that the nucleic acid amplification reaction is delayed.

A sample in an amount of 1 $\mu$L containing a nucleic acid (RNA) (virus; 30,000 copies) was added to 150 $\mu$L of an extraction solution (aqueous solution containing 4 mol/L of urea, 4 mol/L of guanidine hydrochloride, 2 mol/L of calcium chloride, and Tris-HCl Buffer (pH 7.0)) in which nasal mucus was suspended, and the RNA recovery rate was determined in the same manner. The results are shown in Fig. 6. In Examples 3 to 6, it is found that the RNA recovery rate is good even when a sample containing nasal mucus is used.

(Examples 7 to 22 and Comparative Example 6)

In Examples 7 to 22 and Comparative Example 6, a hydrochloric acid-potassium chloride buffer solution having a concentration of 10 mmol/L and a pH of 2.0 was used as the wash solution. In Examples 7 to 13, trishydroxymethylaminomethane (Tris) having a pH shown in Table 2 below and a concentration shown in Table 2 below was used as the recovery solution. In Examples 14 to 17, N-tris(hydroxymethyl)methyl-2-aminoethanesulfonic acid (TES) having a pH shown in Table 2 below and a concentration shown in Table 2 below was used as the recovery solution. In Examples 18 to 21, tricine having a pH shown in Table 2 below and a concentration shown in Table 2 below was used as the recovery solution. In Example 22, TaqPath 1-Step Multiplex MasterMix (1 $\times$ Master Mix, pH 8.0, manufactured by Thermo Fisher Scientific) diluted 4 times was used as the recovery solution. In Comparative Example 6, trishydroxymethylaminomethane (Tris) having a pH of 5.0 and a concentration of 10 mmol/L (10 mM) was used as the recovery solution. The RNA recovery rate and the Ct value were determined in the same manner as in Example 3 except the above. The results are shown in Table 2 below. The recovery rate and the Ct value in Table 2 below are average values of three times of measurement with N = 3.

[Table 2]

|  | Composition of recovery solution | pH of recovery solution | Recovery rate | Ct value |
|---|---|---|---|---|
| Ex. 7 | 10mM Tris | 6.0 | 9.8% | 36.27 |
| Ex. 8 | 10mM Tris | 7.0 | 45.4% | 33.54 |
| Ex. 9 | 10mM Tris | 8.0 | 75.4% | 32.50 |
| Ex. 10 | 10mM Tris | 9.0 | 75.0% | 32.44 |
| Ex. 11 | 5mM Tris | 8.0 | 68.4% | 32.75 |
| Ex. 12 | 50mM Tris | 8.0 | 74.5% | 32.53 |
| Ex. 13 | 100mM Tris | 8.0 | 71.0% | 32.61 |
| Ex. 14 | 5mM TES | 8.0 | 80.2% | 32.30 |
| Ex. 15 | 10mM TES | 8.0 | 73.2% | 32.56 |
| Ex. 16 | 50mM TES | 8.0 | 84.4% | 32.31 |

(continued)

|  | Composition of recovery solution | pH of recovery solution | Recovery rate | Ct value |
|---|---|---|---|---|
| Ex. 17 | 100mM TES | 8.0 | 79.4% | 32.46 |
| Ex. 18 | 5mM Tricine | 8.0 | 76.7% | 32.48 |
| Ex. 19 | lOmM Tricine | 8.0 | 69.5% | 32.68 |
| Ex. 20 | 50mM Tricine | 8.0 | 84.4% | 32.29 |
| Ex. 21 | 100mM Tricine | 8.0 | 63.5% | 32.84 |
| Ex. 22 | TaqPath | 8.1 | 75.3% | 32.51 |
| Comp.Ex.6 | 10mM Tris | 5.0 | 0.0% | No amplification |

As shown in Table 2, it is found that the nucleic acid can be recovered at a good nucleic acid recovery rate in Examples 7 to 22. In Examples 7 to 22, the Ct value was less than 38.0, and a remarkable increase in the Ct value was not observed, which shows that the nucleic acid amplification reaction is hardly inhibited. On the other hand, in Comparative Example 6, the nucleic acid recovery rate is less than 5%, which shows that the nucleic acid recovery rate is insufficient. In addition, in Comparative Example 6, a nucleic acid amplification reaction was not observed as the nucleic acid could not be recovered.

(Examples 23 to 29)

In Examples 23 to 29, a hydrochloric acid-potassium chloride buffer solution having a concentration of 10 mmol/L and a pH of 2.0 was used as the wash solution. In Examples 23 to 29, trishydroxymethylaminomethane (Tris) having a pH of 8.0 and a concentration of 10 mmol/L (10 mM) was used as the recovery solution. In Examples 23 to 29, the metal cations shown in Table 3 below were used. The results are shown in Table 3 below. The recovery rate and the Ct value in Table 3 below are average values of three times of measurement with N = 3.

[Table 3]

|  | Composition of wash solution | pH of wash solution | Composition of recovery solution | pH of recovery solution | Metal cation | Recovery rate | Ct value |
|---|---|---|---|---|---|---|---|
| Ex. 23 | lOmM KCl | 2.0 | lOmM Tris | 8.0 | LiCl | 5.4% | 33.19 |
| Ex. 24 | lOmM KCl | 2.0 | lOmM Tris | 8.0 | NaCl | 45.4% | 29.83 |
| Ex. 25 | lOmM KCl | 2.0 | lOmM Tris | 8.0 | KCl | 30.7% | 30.46 |
| Ex. 26 | lOmM KCl | 2.0 | lOmM Tris | 8.0 | RbCl | 31.2% | 30.38 |
| Ex. 27 | lOmM KCl | 2.0 | lOmM Tris | 8.0 | $CaCl_2$ | 56.2% | 30.38 |
| Ex. 28 | lOmM KCl | 2.0 | lOmM Tris | 8.0 | $MgCl_2$ | 67.8% | 29.17 |
| Ex. 29 | lOmM KCl | 2.0 | lOmM Tris | 8.0 | $SrCl_2$ | 73.7% | 29.00 |

As shown in Table 3, it is found that the nucleic acid can be recovered at an improved recovery rate when divalent $CaCl_2$, $MgCl_2$, or $SrCl_2$ is used as the metal cation.

(Reference Example)

In Reference Example, RNA recovery rates were compared and examined when NaCl (sodium ion), $CaCl_2$

(calcium ion), MgCl$_2$ (magnesium ion), KCl (potassium ion), LiCl (lithium ion), and RbCl (rubidium ion) were used as the metal cation (metal cation for RNA adsorption).

The recovery rates were measured as follows using the same chip 1 as in Example 1.

First, 1 μL of a sample containing a nucleic acid (RNA) (virus; 5000 copies) was added to 60 μL of a nucleic acid extract (aqueous solution containing 2 mol/L of metal cation and 50 mmol/LTris-HCl Buffer (pH 8.0)) to extract the nucleic acid.

Next, the mixed solution after the nucleic acid extraction was sent from the upstream flow path 2a to the recovery part 3 to cause the nucleic acid to adsorb to the silica fibers of the recovery part 3.

Next, 200 μL of a wash solution was sent to the recovery part 3 to wash the silica fibers carrying the nucleic acid. Subsequently, the recovery part 3 was heated by a heater at 80°C for 3 minutes to dry the wash solution. The washing was performed twice, that is, washing with 1.8 mol/L guanidine thiocyanate, 40% ethanol, and 30 mmo/L Tris-HCl (pH 7.0) and washing with a wash solution containing 90% ethanol were performed. After the wash solution was removed, 28 μL of a recovery solution (1 × Master Mix) was sent to the recovery part 3, and the nucleic acid (RNA) supported by the recovery part 3 was isolated and recovered.

The results are shown in Fig. 9. As is apparent from Fig. 9, it is found that the recovery rate is further increased when divalent CaCl$_2$ or MgCl$_2$ is used as the metal cation.

## EXPLANATION OF SYMBOLS

| | |
|---|---|
| 1: | Chip |
| 2: | Flow path |
| 2a: | Upstream flow path |
| 2b: | Downstream flow path |
| 3: | Recovery part |
| 4: | Anionic adsorbent |
| 5: | Substrate |
| 5a: | Main face |
| 5b: | Recess |
| 6: | Cover member |
| 7a: | Injection port |
| 7b: | Recovery port |

## Claims

1. A method for purifying a nucleic acid, the method comprising:

    a step of bringing a sample containing a nucleic acid into contact with an extraction solution containing a metal cation to prepare an extraction solution containing the nucleic acid;
    a step of bringing the extraction solution containing the nucleic acid into contact with an anionic adsorbent to cause the nucleic acid to adsorb to the anionic adsorbent;
    a step of bringing a wash solution having a pH of 5.0 or less into contact with the anionic adsorbent to wash the anionic adsorbent to which the nucleic acid is adsorbed; and
    a step of bringing a recovery solution having a pH of 6.0 or more into contact with the anionic adsorbent to isolate the nucleic acid from the anionic adsorbent.

2. The method for purifying a nucleic acid according to claim 1, wherein the recovery solution is a solution that does not inhibit a nucleic acid amplification reaction.

3. The method for purifying a nucleic acid according to claim 1 or 2, wherein the recovery solution is a buffer solution.

4. The method for purifying a nucleic acid according to claim 3, wherein a concentration of the buffer solution is 5 mmol/L or more and 100 mmol/L or less.

5. The method for purifying a nucleic acid according to claim 1 or 2, wherein the recovery solution is a solution containing a nucleic acid amplification reagent.

**6.** The method for purifying a nucleic acid according to any one of claims 1 to 5, wherein the wash solution having a pH of 5.0 or less is at least one selected from the group consisting of water, a hydrochloric acid-potassium chloride buffer solution, a glycine-hydrochloric acid buffer solution, a citric acid-sodium citrate buffer solution, and a citric acid-phosphate buffer solution.

**7.** The method for purifying a nucleic acid according to any one of claims 1 to 6, wherein a concentration of the metal cation in the extraction solution is 0.5 mol/L or more.

**8.** The method for purifying a nucleic acid according to any one of claims 1 to 7, wherein the metal cation is an alkali metal ion, an alkaline earth metal ion, a transition metal ion, or a group 12 metal ion.

**9.** The method for purifying a nucleic acid according to any one of claims 1 to 8, wherein the metal cation has a valence of 2 or more.

[FIG. 1.]

[FIG. 2.]

<u>1</u>

[FIG. 3.]

<u>1</u>

[FIG. 4.]

[FIG. 5.]

[FIG. 6.]

[FIG. 7.]

[FIG. 8.]

[FIG. 9.]

## INTERNATIONAL SEARCH REPORT

| International application No. |
| --- |
| PCT/JP2021/019014 |

### A. CLASSIFICATION OF SUBJECT MATTER
C12N 15/10(2006.01)i
FI: C12N15/10 Z

According to International Patent Classification (IPC) or to both national classification and IPC

### B. FIELDS SEARCHED

Minimum documentation searched (classification system followed by classification symbols)
C12N15/10

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2012-255798 A (F.HOFFMANN-LA ROCHE AG) 27 December 2012 (2012-12-27) paragraphs [0001], [0028]-[0029], [0062], [0065]-[0066] | 1-9 |
| A | JP 2015-62406 A (EIKEN CHEMICAL CO., LTD.) 09 April 2015 (2015-04-09) | 1-9 |
| A | CN 102146369 A (JILIN UNIVERSITY) 10 August 2011 (2011-08-10) | 1-9 |
| P, X | WO 2020/153248 A1 (SEKISUI CHEMICAL CO., LTD.) 30 July 2020 (2020-07-30) claims, examples | 1-9 |

☐ Further documents are listed in the continuation of Box C.　　☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 20 July 2021 (20.07.2021) | 10 August 2021 (10.08.2021) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2021/019014

| Patent Documents referred in the Report | Publication Date | Patent Family | Publication Date |
|---|---|---|---|
| JP 2012-255798 A | 27 Dec. 2012 | US 2008/0319182 A1 paragraphs [0002], [0028]-[0029], [0062], [0065]-[0066] EP 1983051 A2 CA 2629589 A CN 101289661 A HK 1125672 A CA 2629589 A1 | |
| JP 2015-62406 A | 09 Apr. 2015 | (Family: none) | |
| CN 102146369 A | 10 Aug. 2011 | (Family: none) | |
| WO 2020/153248 A1 | 30 Jul. 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018134093 A

- JP 4340298 B